# EUROPEAN PATENT SPECIFICATION

(11) **EP 2 006 565 B1**
(45) Date of publication and mention of the grant of the patent: **23.10.2013**
(21) Application number: 08010886.3
(22) Date of filing: 16.06.2008
(51) Int. Cl.: F16D 63/00, F16D 65/14, A61B 1/005

(54) **Gear apparatus**
Getriebevorrichtung
Appareil à engrenage

(30) Priority: 19.06.2007 JP 2007161485
(43) Date of publication of application: 24.12.2008
(73) Proprietor: Olympus Medical Systems Corp., Tokyo 151-0072 (JP)
(72) Inventor: Masaki, Yutaka, Hachioji-shi Tokyo 192-8512 (JP)
(74) Representative: von Hellfeld, Axel

(56) References cited:
- DE-U1-202005 015 052
- JP-A- 2006 212 357
- US-A- 6 125 983
- US-A1- 2007 004 967

## Description

The present invention relates to a gear apparatus to limit the rotation of a spur gear.

Various gear apparatuses to limit the rotation of a spur gear are used.

In a gear apparatus of Jpn. Pat. Appln. KOKAI Publication No. 2006-212357 , a rotation limitation member is moved toward a spur gear, an engaging portion of the limitation member is engaged with the teeth of the spur gear, and so the rotation of the spur gear is limited.

In a gear apparatus wherein a limitation member is moved toward a spur gear, teeth of the limitation member are engaged with the teeth of the spur gear and so the rotation of the gear is limited, when the tip of the tooth of the limitation member contacts the tip of the tooth of the spur gear, the teeth of the limitation member can not be engaged with the teeth of the spur gear, and so an operational failure may occur in the gear apparatus.

US 6,125,983 discloses a parking sprag for engaging a parking gear of an automotive transmission. An electric motor drives a linkage cam which drives the linkage assembly in order to engage the sprag mechanism with the parking gear. If the sprag member comes into contact with a top portion of a tooth of the parking gear, it will not be allowed to engage the parking gear. The cam is further rotated, causing further rotation of a lever connected to springs which control the rotation of the sprag member. As the spring force builds, the sprag member tooth is strongly urged against the tooth of the parking gear. When the parking gear continues to rotate after the sprag is forced against the parking gear, the sprag member engages the teeth of the parking gear.

DE 20 2005 015 052 discloses a rear brake mechanism activated by pressing a pedal to rotate a tie bar, thereby rotating a cam-like mechanism and forcing a braking pin forward. The pin has a spring creating a load between the head of the pin and a wall of a tube. The tube has a spring at its opposite end loaded between the tube end wall and the inside wall of a surrounding tube. A protrusion extends longitudinally along the outside of the tube and is forced forward when the cam activates the braking pin. The protrusion brakes wheels by extending forward into a space between two ribs located on the wheel hubs.

The present invention has been made in view of the foregoing problem, and is directed to provide a compact gear apparatus wherein an operational failure is prevented.

This problem is solved by a gear apparatus as defined in claim 1.

The gear apparatus according to the invention comprises: a spur gear including teeth and to be rotated around a rotation axis; a rotation mechanism to rotate the spur gear; a limitation member including tooth to be engaged with the teeth of the spur gear, the limitation member to limit the rotation of the spur gear; a movement mechanism to move the limitation member in the radial direction of the rotation axis such that the tooth of the limitation member is engaged with the teeth of the spur gear; an input mechanism to input power to actuate the movement mechanism; a transmission mechanism to transmit the power input by the input mechanism to the movement mechanism; and an accumulation mechanism provided in the transmission mechanism and to accumulate the power input by the input mechanism when a tip of the tooth of the limitation member contacts a tip of the tooth of the spur gear.

In the gear apparatus, while the limitation member is moved toward the spur gear, if the tip of the tooth of the limitation member contacts the tip of the tooth of the spur gear, the movement of the limitation member is once stopped, and the input power is accumulated. Then, when the spur gear is rotated and the phase of the tip of the tooth of the limitation member corresponds to the phase of a root between the teeth of the spur gear, the limitation member is again moved by the accumulated power, and so the limitation member is engaged with the spur gear. Therefore, an operational failure in the gear apparatus is prevented.

In the gear apparatus the accumulation mechanism includes an elastic member.

In the gear apparatus, the power is accumulated as elastic energy.

The gear apparatus of present invention is characterised in that the input mechanism includes an input member to be rotated about a rotation axis which is coaxial with the rotation axis of the spur gear. The movement mechanism includes a movement member to be rotated about a rotational axis coaxial with the rotation axis of the input member and the spur gear. The elastic member is provided between the input member and the movement member and is designed to be deformed by the rotation of the input member to produce urging force to rotate the movement member in the same direction as the rotational direction of the input member.

In the gear apparatus, when the input member is rotated, the movement member is rotated via the elastic member. If the tip of the tooth of the limitation member contacts the tip of the tooth of the spur gear, the rotation of the movement member is once stopped, and the power is accumulated by the deformation of the elastic member. Then, when the spur gear is rotated and the phase of the tip of the tooth of the limitation member corresponds to the phase of the root between the teeth of the spur gear, the movement member is again rotated by the reset of the elastic member.

In a preferred aspect of the present invention, the gear apparatus is characterized by further comprising: a positioning mechanism to position the spur gear so that the phase of a root between the teeth of the spur gear corresponds to the phase of the tip of the tooth of the limitation member.

In the gear apparatus, the tip of the tooth of the limitation member is prevented from contacting the tip of the tooth of the spur gear, and so an operational failure in the gear apparatus is prevented.

In a preferred aspect of the present invention, the gear apparatus is characterized in that the positioning mechanism includes a lock member to be locked between the teeth of the spur gear, and an urging member to urge the lock member inwardly in the radial direction of the rotation axis.

In the gear apparatus, the engaging member is urged by the urging member and engaged between the teeth of the spur gear, and so the spur gear is positioned.

The invention can be more fully understood from the following detailed description when taken in conjunction with the accompanying drawings, in which:
FIG. 1 is a perspective view showing an endoscope system in a first embodiment of the present invention;
FIG. 2 is a schematic diagram showing a power transmission mechanism in the first embodiment of the present invention;
FIG. 3 is a schematic diagram showing the power transmission mechanism in the first embodiment of the present invention along the III-III line of FIG. 2;
FIG. 4 is a perspective view showing a clutch mechanism in the first embodiment of the present invention;
FIG. 5 is a sectional view showing the clutch mechanism in the first embodiment of the present invention cut along the V-V line of FIG. 4;
FIG. 6 is an exploded perspective view showing the clutch mechanism in the first embodiment of the present invention;
FIG. 7A is a schematic diagram showing the clutch mechanism in a released state in the first embodiment of the present invention;
FIG. 7B is a sectional view showing the clutch mechanism in the released state in the first embodiment of the present invention;
FIG. 8A is a schematic diagram showing the clutch mechanism in a stopped state in the first embodiment of the present invention;
FIG. 8B is a sectional view showing the clutch mechanism in the stopped state in the first embodiment of the present invention;
FIG. 9A is a schematic diagram showing the clutch mechanism in a connected state in the first embodiment of the present invention;
FIG. 9B is a sectional view showing the clutch mechanism in the connected state in the first embodiment of the present invention;
FIG. 10 is a front view showing a clutch mechanism in a first modification of the first embodiment of the present invention;
FIG. 11 is a sectional view showing the clutch mechanism in the first modification of the first embodiment of the present invention;
FIG. 12 is a front view showing a clutch mechanism in a second modification of the first embodiment of the present invention;
FIG. 13 is a sectional view showing the clutch mechanism in the second modification of the first embodiment of the present invention;
FIG. 14 is a sectional view showing a positioning mechanism in a second embodiment of the present invention;
FIG. 15 is a schematic diagram showing a clutch mechanism in a first referential embodiment of the present invention;
FIG. 16 is a schematic diagram for explaining the operation of the clutch mechanism in the first referential embodiment of the present invention;
FIG. 17 is a schematic diagram showing a clutch mechanism in a second referential embodiment of the present invention; and
FIG. 18 is a schematic diagram showing a clutch mechanism in a third referential embodiment of the present invention.

Embodiments of the present invention will hereinafter be described with reference to the drawings.

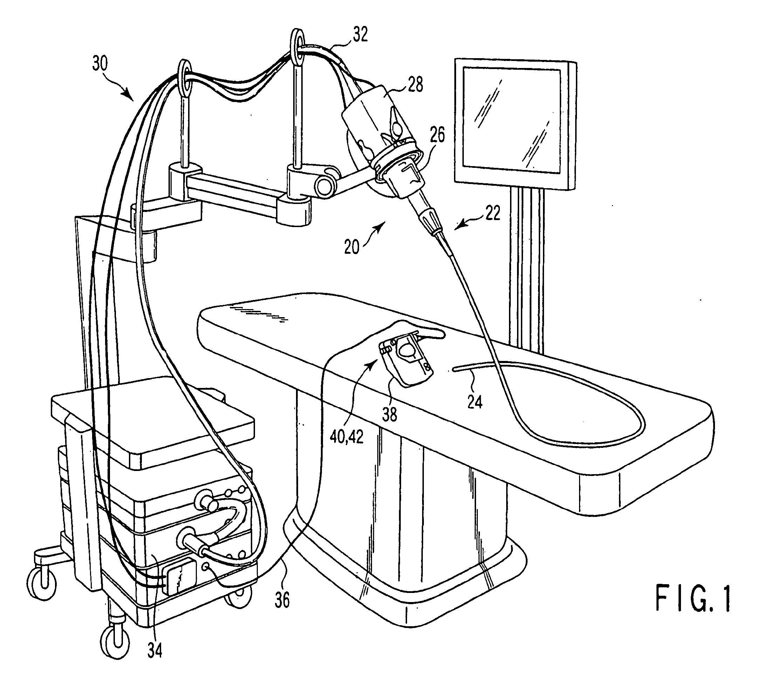

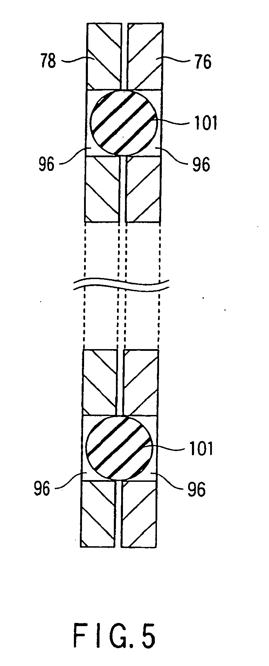

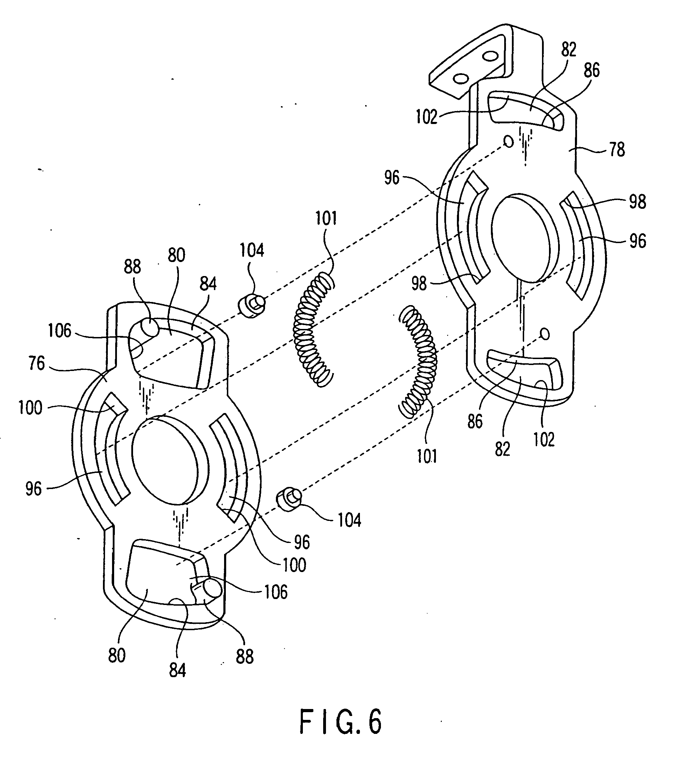

FIGS. 1 to 9B show a first embodiment of the present invention.

An electric bending endoscope 20 (hereinafter simply referred to as an endoscope 20) of an endoscope system includes an elongate insertion portion 22 to be inserted into a body cavity. A bending portion 24 to be operated to be bent is provided at the distal end of the insertion portion 22, and an insertion and removal portion 26 is provided at the proximal end of the insertion portion 22. Here, the insertion and removal portion 26 includes an angle mechanism therein, and an angle wire extending out of the angle mechanism is inserted through the insertion portion 22, and coupled to the distal end of the bending portion 24. Further, the insertion and removal portion 26 is removably inserted into a motor unit 28, and a drive apparatus for actuating the angle mechanism is provided in the motor unit 28. As described later, a clutch mechanism is provided in the drive apparatus and the drive apparatus functions as a gear apparatus. The motor unit 28 is held by a holding apparatus 30 such that the motor unit 28 is movable and fixable, and rotatable about its central axis. Moreover, the motor unit 28 is connected to a video processor 34 via a universal cord 32, and an operation portion 38 to be held and operated by an operator is connected to the video processor 34 via an electric cord 36. The operation portion 38 is provided with a changeover switch 40 and a bending switch 42. When the changeover switch 40 is switched between a connection position and a release position, the clutch mechanism of the drive apparatus is switched between a connected state and a released state. When the bending switch 42 is operated, the angle mechanism is actuated by the drive apparatus of the motor unit 28, the angle wire is moved back and forth, and so the bending portion 24 is bend.

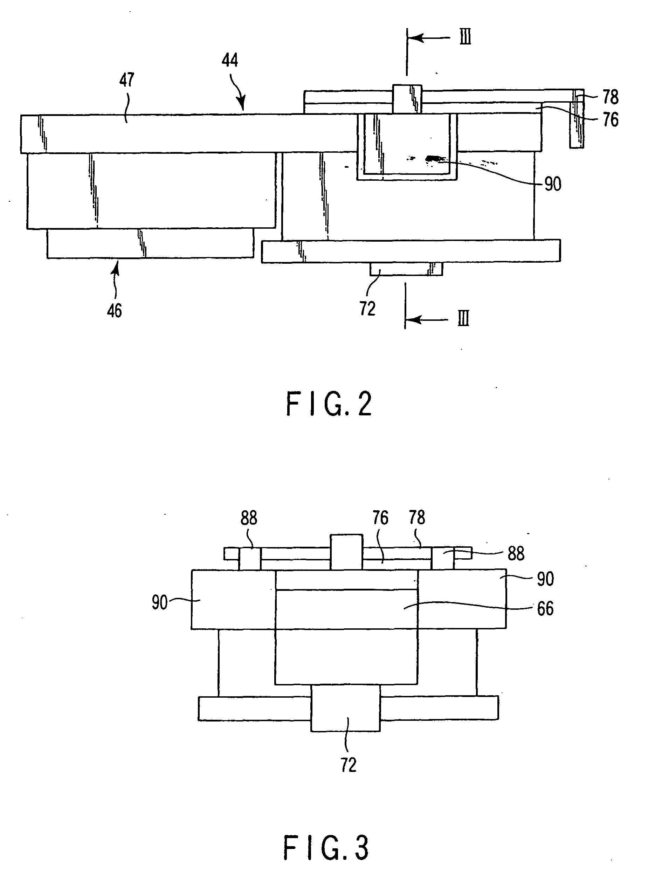

The drive apparatus functioning as the gear apparatus will be described with reference to FIGS. 2 to 6.

A power transmission mechanism 44 is explained with reference to FIGS. 2 and 3.

A drive shaft of a motor 46 is connected to an output shaft 72 at a reduction gear ratio via gear train in a gear unit 47. Here, a fixing gear 66 as an annular gear is interposed between the gear train. The fixing gear 66 is switchable between a fixing state unrotatable about its central axis and a fixing-released state rotatable. In the case where the fixing gear 66 is in the fixing state, when the drive shaft of the motor 46 is rotated, the gear train are sequentially rotated and the output shaft 72 is rotated at a reduced rotation velocity. On the other hand, in the case where the fixing gear 66 is in the fixing-released state, even when the drive shaft of the motor 46 is rotated, the gear train idles and rotation torque is not transmitted to the output shaft 72.

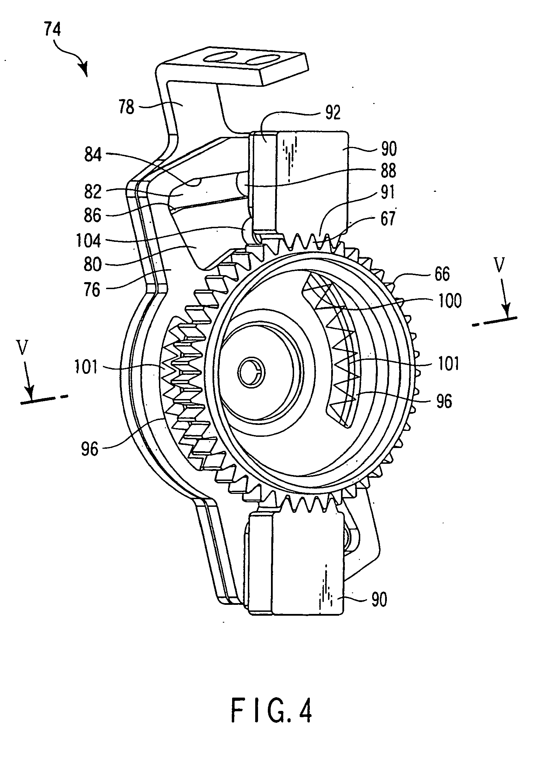

A clutch mechanism 74 of the drive apparatus is explained with reference to FIGS. 4 to 6.

In the clutch mechanism 74, the fixing gear 66, a connection cam 76 and a release cam 78 are sequentially provided in the direction of the rotational axis of the fixing gear 66. The connection cam 76 and the release cam 78 are in the shape of long plate perpendicular to the rotational axis of the fixing gear 66, and rotatable between a connection position and a release position about a rotational axis coaxial with the rotational axis of the fixing gear 66. Connection guide holes 80 penetrate at both ends of the connection cam 76 in symmetry with respect to the rotational axis, and release guide holes 82 penetrate at both ends of the release cam 78 in symmetry with respect to the rotational axis. A connection cam face 84 is formed by the end side surface defining the connection guide hole 80 in the connection cam 76, while a release cam face 86 is formed by the rotational axis side surface defining the release guide hole 82 in the release cam 78. A cam pin 88 is inserted through the connection guide hole 80 of the connection cam 76 and the release guide hole 82 of the release cam 78, and the cam pin 88 protrudes from a limitation member 90 disposed radially outside the fixing gear 66. Both side surfaces of the limitation member 90 forms slide surfaces 92, slide surfaces 92 are supported slidably in the radial direction of the fixing gear 66, and the limitation member 90 is movable in the radial direction of the fixing gear 66.

In the case where the connection cam 76 and the release cam 78 are integrally rotated in phase, when the connection cam 76 and the release cam 78 are rotated from the release position to the connection position, the cam pin 88 slid along the connection cam face 84 on the end side in the connection cam 76, and so the limitation member 90 is moved to the radially inside connection position inwardly in the radial direction of the fixing gear 66. When the connection cam 76 and the release cam 78 are rotated from the connection position to the release position, the cam pin 88 slid along the release cam face 86 on the rotational axis side in the release cam 78, and so the limitation member 90 is moved to the radially outside release position outwardly in the radial direction of the release cam 78.

Teeth 91 to engage with external teeth 67 of the fixing gear 66 are formed in a radially inner portion of the limitation member 90. When the limitation member 90 is at the connection position, the teeth 91 of the limitation member 90 are engaged with the external teeth 67 of the fixing gear 66, and the fixing gear 66 is brought into an unrotatable fixing state by the limitation member 90 which is circumferentially unrotatable. On the other hand, when the limitation member 90 is at the release position, the teeth 91 of the limitation member 90 are separated from the external teeth 67 of the fixing gear 66, and the fixing gear 66 is brought into the rotatable fixing-released state.

When the fixing gear 66 is in the fixing state, the transmission of power by the power transmission mechanism 44 is possible. This is the connected state of the clutch mechanism 74. When the fixing gear 66 is in the fixing-released state, the respective gears idle and so the transmission of power by the power transmission mechanism 44 is impossible. This is the released state of the clutch mechanism 74.

The configuration of the drive apparatus as the gear apparatus is explained with reference to FIGS. 4 to 6.

As described above, a cam mechanism is used as a movement mechanism, and when the connection cam 76 as a movement member is rotated from the release position to the connection position, the limitation member 90 is moved inwardly in the radial direction of the fixing gear 66, and the teeth 91 of the limitation member 90 are engaged with the external teeth 67 of the fixing gear 66. Moreover, when the limitation member 90 is not engaged with the fixing gear 66, the driving force of the motor 46 of the drive apparatus is cut off, and the fixing gear 66 and the output shaft 72 is freely rotatable independent of the driving force of the motor 46.

The release cam 78 as an input member is electrically rotatable from the release position to the connection position.

A pair of receiving holes 96 penetrates in each of the release cam 78 and the connection cam 76, and extends in the circumferential direction of the rotational axis in symmetry with respect to the rotational axis. When the release cam 78 and the connection cam 76 are in phase, the receiving hole 96 of the release cam 78 and the receiving hole 96 of the connection cam 76 are disposed to overlap each other. An input side receiving surface 98 is formed at the releasing direction side end of the receiving hole 96 of the release cam 78, and output side receiving surface 100 is formed at the connecting direction side end of the receiving hole 96 of the connection cam 76. An compression spring 101 as a elastic member is received in the overlapped receiving holes 96 of the release cam 78 and the receiving hole 96 of the connection cam 76, and one end of the compression spring 101 is supported by the input side receiving surface 98 of the release cam 78, while the other end thereof is supported by the output side receiving surface 100 of the connection cam 76.

In the release cam 78, a clearance surface 102 is formed by the end side surface defining the release guide hole 82 in opposition to the rotational axis side release cam face 86.

In addition, a drive pin 104 protrudes from a connection cam 76 side surface of the release cam 78. The drive pin 104 is inserted into the connection guide hole 80 of the connection cam 76. When the release cam 78 and the connection cam 76 is in phase, the drive pin 104 contacts a drive surface 106 formed by a releasing direction side surface of the connection guide hole 80.

Next, the actuation of the drive apparatus as the gear apparatus in the present embodiment will be described.

When the bending portion 24 of the endoscope 20 is bent, the changeover switch 40 is switched from the release position to the connection position in order to switch the clutch mechanism 74 from the released state to the connected state, and then the bending switch 42 is operated in order to bend the bending portion 24.

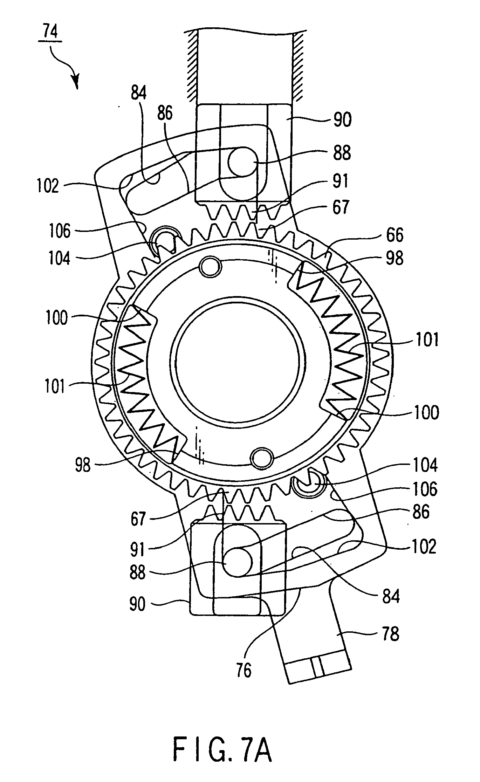

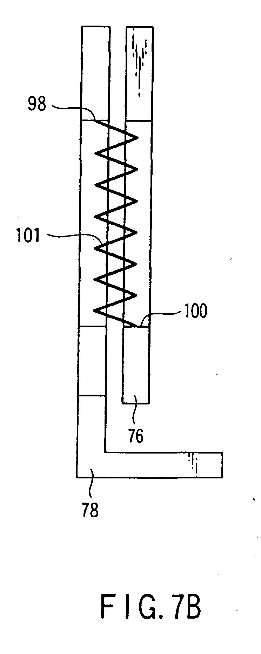

As shown in FIGS. 7A and 7B, when the clutch mechanism 74 is in the released state, the release cam 78 and the connection cam 76 are in phase and in the release position, and the limitation member 90 is in the release position, and the fixing gear 66 is rotatable. When the changeover switch 40 is switched from the release position to the connection position, the release cam 78 is electrically rotated from the release position to the connection position. When the release cam 78 is rotated, the connection cam 76 is pressed by the slightly compressed and deformed compression spring 101 and thus rotated in the same direction as the rotational direction of the release cam 78, and the cam pin 88 is slid on the end side connection cam face 84 in the connection cam 76, and so the limitation member 90 is moved inwardly in the radial direction of the fixing gear 66.

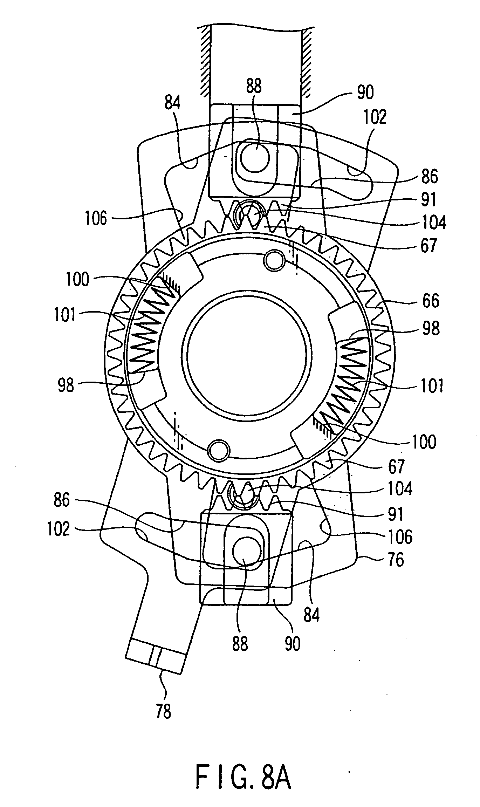

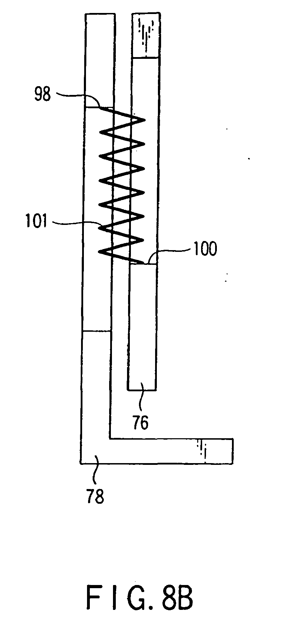

As shown in FIGS. 8A and 8B, when the phase of the tip of the tooth 91 of the limitation member 90 corresponds to the phase of the tip of the external tooth 67 of the fixing gear 66, the tip of the tooth 91 of the limitation member 90 contacts the tip of the external tooth 67 of the fixing gear 66. In this case, the release cam 78 keeps rotation to the connection position, but the movement of the limitation member 90 and the rotation of the connection cam 76 are once stopped, the compression spring 101 between the connection cam 76 and the release cam 78 is compressed and deformed, and so the power is accumulated as elastic energy. In addition, while the connection cam 76 is being stopped and the release cam 78 is rotating, there is no interference between the release cam 78 and the cam pin 88 owing to the function of the end side clearance surface 102 in the release cam 78. In this manner, while the release cam 78 is disposed in the connection position, the limitation member 90 and the connection cam 76 are stopped at a stop position between the release position and the connection position, and the fixing gear 66 remains rotatable.

Then, when the bending switch 42 is operated, the motor 46 of the drive apparatus is driven. As the clutch mechanism 74 is not in the connected state, the respective gears idle, and the fixing gear 66 is also rotated. Further, when the phase of the tip of the tooth 91 of the limitation member 90 corresponds to the phase of a root between the external tooth 67 of the fixing gear 66, the stopping of the limitation member 90 and the connection cam 76 is released, the compressed and deformed compression spring 101 reset, and so the power is input to the connection cam 76, and the connection cam 76 is rotated in the same direction as the rotational direction of the release cam 78.

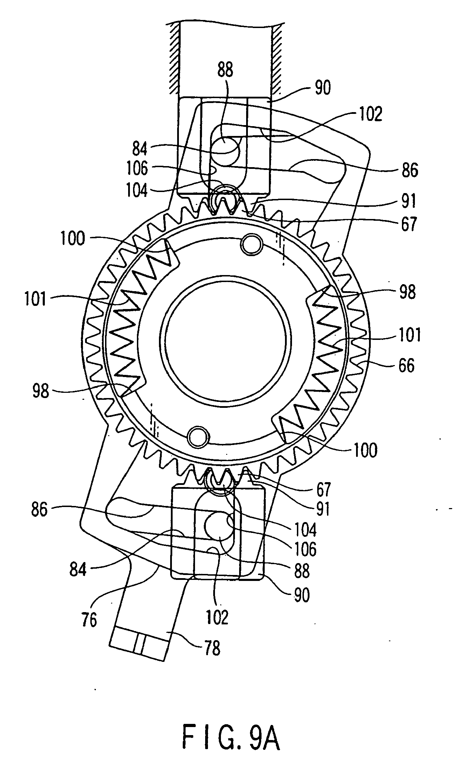

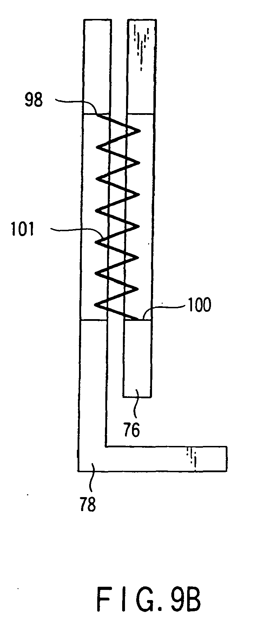

As shown in FIGS. 9A and 9B, as the connection cam 76 is rotated, the cam pin 88 is slid on the end side connection cam face 84 in the connection cam 76, the limitation member 90 is radially inwardly moved to the connection position, the limitation member 90 is engaged with the fixing gear 66, and the fixing gear 66 is fixed. When the fixing gear 66 is fixed, the transmission of power by the power transmission mechanism 44 is enabled, the angle mechanism is actuated by the drive apparatus, and the bending portion 24 is bent.

On the other hand, when the phase of the tip of the tooth 91 of the limitation member 90 corresponds to the phase of the root between the external teeth 67 of the fixing gear 66, the release cam 78 and the connection cam 76 are integrally rotated to the connection position substantially in phase with each other, and the limitation member 90 is moved to the connection position without being stopped, and so the limitation member 90 is engaged with the fixing gear 66.

In addition, when an operator of the endoscope 20, for example, a medical doctor judges that the bending portion 24 needs to reset into a linear state, the changeover switch 40 is switched from the connection position to the release position, and so the release cam 78 is electrically rotated from the connection position to the release position. Consequently, as shown from FIGS. 9A and 9B to FIGS. 7A and 7B, the drive pin 104 of the release cam 78 contacts the releasing direction side drive surface 106 in the connection cam 76 and drives the connection cam 76, and the connection cam 76 is rotated from the connection position to the release position integrally with the release cam 78. Then, the cam pin 88 is slid along the end side release cam face 86 in the release cam 78, the limitation member 90 is moved from the connection position to the release position, the engagement of the fixing gear 66 and the limitation member 90 is released, and the fixing of the fixing gear 66 is released. When the fixing of the fixing gear 66 is released, the transmission of power by the power transmission mechanism 44 is impossible, and the angle mechanism is free, and the bending portion 24 reset to the linear state.

Therefore, the drive apparatus as the gear apparatus in the present embodiment includes the following effect.

In the drive apparatus of the present embodiment, when the release cam 78 is rotated, the connection cam 76 is rotated via the compression spring 101, and the limitation member 90 is thus moved toward the fixing gear 66. When the tip of the tooth 91 of the limitation member 90 contacts the tip of the external tooth 67 of the fixing gear 66, the movement of the limitation member 90 and the rotation of the connection cam 76 are once stopped, and the power is accumulated as elastic energy by the compression and deformation of the compression spring 101. Then, when the fixing gear 66 is rotated and the phase of the tip of the tooth 91 of the limitation member 90 corresponds to the phase of the root between the external teeth 67 of the fixing gear 66, the connection cam 76 is again rotated by the reset of the compression spring 101, the limitation member 90 is again moved, and the limitation member 90 is engaged with the fixing gear 66. Therefore, an operational failure in the gear apparatus is prevented.

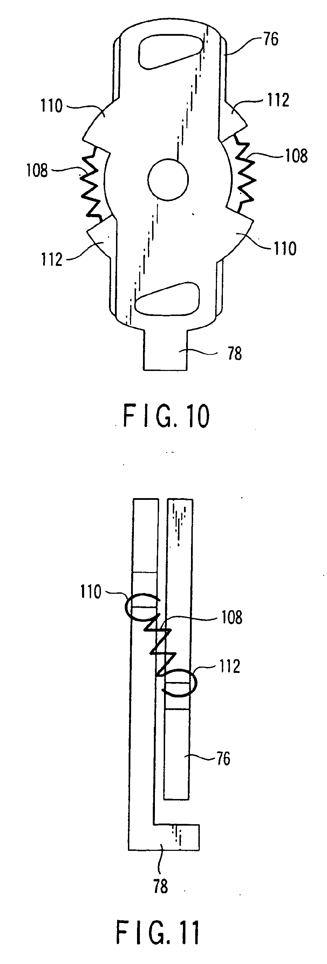

FIGS. 10 and 11 show a first modification of the first embodiment of the present invention.

In the present modification, a tension spring 108 is used as an elastic member. An input side coupling portion 110 is formed in the release cam 78 and one end of the tension spring 108 is coupled to the input side coupling portion 110. An output side coupling portion 112 is formed in the connection cam 76 and the other end of the tension spring 108 is coupled to the output side coupling portion 112. The input side coupling portion 110 and the output side coupling portion 112 are disposed on the same circumference with respect to the rotational axis of the release cam 78 and the connection cam 76. When the release cam 78 and the connection cam 76 is in phase, the input side coupling portion 110 is disposed on the connecting direction side and the output side coupling portion 112 is disposed on the releasing direction side.

When the release cam 78 is electrically rotated, the connection cam 76 is pulled by the slightly pulled and deformed tension spring 108 and thus rotated in the same direction as the rotational direction of the release cam 78. When the tip of the tooth 91 of the limitation member 90 contacts the tip of the external tooth 67 of the fixing gear 66, as in the first embodiment, the release cam 78 keeps rotation to the connection position, but the movement of the limitation member 90 and the rotation of the connection cam 76 are once stopped, and the tension spring 108 between the connection cam 76 and the release cam 78 is pulled and deformed, and power is accumulated as elastic energy. When the phase of the tip of the tooth 91 of the limitation member 90 corresponds to the phase of the root between the external teeth 67 of the fixing gear 66, the stopping of the limitation member 90 and the connection cam 76 is released, the pulled and deformed tension spring 108 resets, the power is input to the connection cam 76, and the connection cam 76 is rotated in the same direction as the rotational direction of the release cam 78.

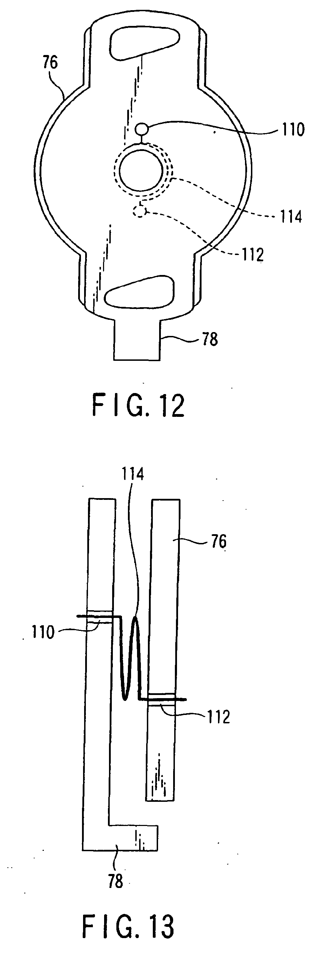

FIGS. 12 and 13 show a second modification of the first embodiment of the present invention.

In the present modification, a torsion spring 114 is used as an elastic member. As in the first modification, the input side coupling portion 110 is formed in the release cam 78, and the output side coupling portion 112 is formed in the connection cam 76.

When the release cam 78 is electrically rotated, the connection cam 76 is urged by the slightly twisted and deformed torsion spring 114 and thus rotated in the same direction as the rotational direction of the release cam 78. When the tip of the tooth 91 of the limitation member 90 contacts the tip of the external tooth 67 of the fixing gear 66, the torsion spring 114 between the connection cam 76 and the release cam 78 is twisted and deformed, and so power is accumulated as elastic energy. When the phase of the tip of the tooth 91 of the limitation member 90 corresponds to the phase of the root between the external teeth 67 of the fixing gear 66, the twisted and deformed torsion spring 114 resets, and so the power is input to the connection cam 76.

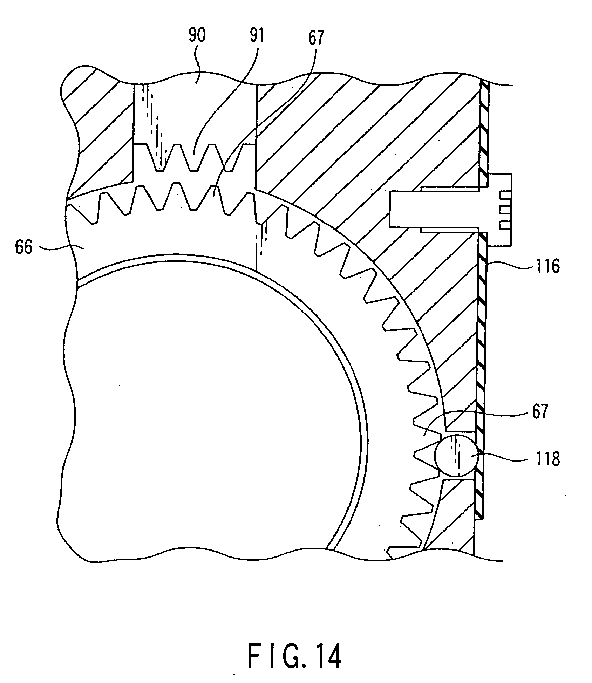

FIG. 14 shows a second embodiment of the present invention.

In the drive apparatus of the present embodiment, the fixing gear 66 is positioned in advance such that the phase of the tip of the tooth 91 of the limitation member 90 corresponds to the phase of the root between the external teeth 67 of the fixing gear 66 in order to prevent the tip of the tooth 91 of the limitation member 90 from contacting the tip of the external tooth 67 of the fixing gear 66.

That is, one end of a leaf spring 116 as an urging member is fixed to a housing, and a spherical member 118 as a lock member is provided at the other end of the leaf spring 116. The spherical member 118 is inserted and locked between the external teeth 67 of the fixing gear 66 by urging force of the leaf spring 116. The limitation member 90 is disposed such that the phase of the tip of the tooth 91 corresponds to the phase of the root between the external teeth 67 of the fixing gear 66 when the spherical member 118 is locked between the external teeth 67 of the fixing gear 66 and thus the fixing gear 66 is positioned.

In addition, when the engagement of the fixing gear 66 and the limitation member 90 is being released, the fixing gear 66 is rotatable by the rotation of the motor 46 of the drive apparatus regardless of the urging of the spherical member 118 toward the fixing gear 66 by the leaf spring 116. That is, when the motor 46 of the drive apparatus is driven, the fixing gear 66 is rotated while the spherical member 118 is pushed out by the external teeth 67 of the fixing gear 66 and the spherical member 118 falls between the external teeth 67 of the fixing gear 66 by the urging of the leaf spring 116, and these are repeated.

In the gear apparatus of the present embodiment, the tip of the tooth 91 of the limitation member 90 is prevented from contacting the tip of the external tooth 67 of the fixing gear 66, and so an operational failure in the gear apparatus is prevented.

Referential embodiments of the present invention will now be described.

In the referential embodiments of the present invention, the fixing gear 66 is rotated by the movement of the limitation member 90, and so the phase of the tip of the tooth 91 of the limitation member 90 corresponds to the phase of the root between the external teeth 67 of the fixing gear 66.

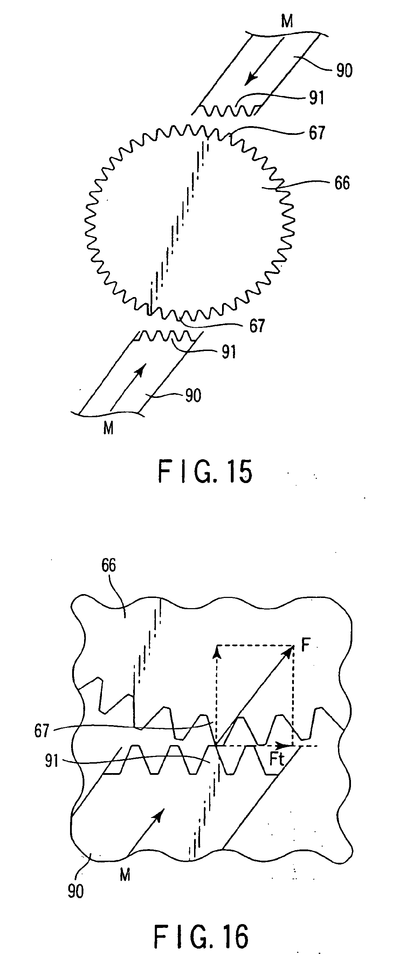

FIGS. 15 and 16 show a first referential embodiment of the present invention.

Referring to FIG. 15, a movement direction M of the limitation member 90 does not pass through the rotation axis of the fixing gear 66, and the rotation axis of the fixing gear 66 is disposed eccentrically with respect to the movement direction M of the limitation member 90. Moreover, in the limitation member 90, the teeth 91 are provided side by side in parallel with the tangential direction of the fixing gear 66.

Referring to FIG. 16, when the limitation member 90 is moved toward the fixing gear 66, if the teeth 91 of the limitation member 90 contact the external teeth 67 of the fixing gear 66, press force F in the movement direction M of the limitation member 90 acts from the teeth 91 of the limitation member 90 to the external teeth 67 of the fixing gear 66. Due to component force Ft of the press force F, in the tangential direction of the fixing gear 66, the fixing gear 66 is rotated, and so the phase of the root between the external teeth 67 of the fixing gear 66 corresponds to the phase of the tip of the tooth 91 of the limitation member 90. As the limitation member 90 is further moved, the teeth 91 of the limitation member 90 are engaged with the external teeth 67 of the fixing gear 66.

As described above, the fixing gear 66 is rotated by the movement of the limitation member 90, the phase of the tip of the tooth 91 of the limitation member 90 corresponds to the phase of the root between the external teeth 67 of the fixing gear 66, and the limitation member 90 is engaged with the fixing gear 66. Therefore, an operational failure in the gear apparatus is prevented.

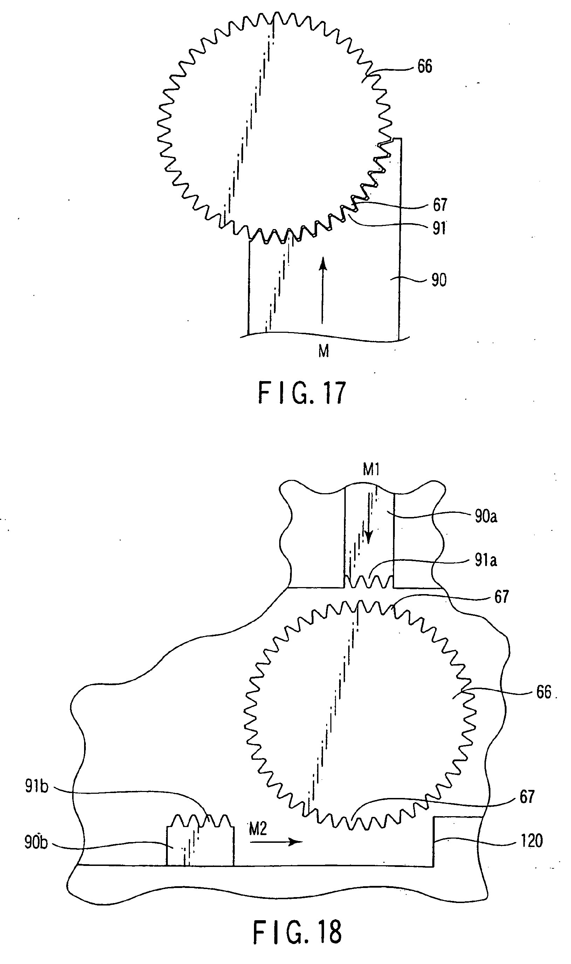

FIG. 17 shows a second referential embodiment of the present invention.

In the present referential embodiment, as the limitation member 90, a limitation member 90 to be engaged with the fixing gear 66 over quarter circumference of the fixing gear 66 is used.

FIG. 18 shows a third referential embodiment of the present invention.

In the present referential embodiment, first and second limitation members 90a, 90b are used. A movement direction M1 of the first limitation member 90a is the radial direction of the fixing gear 66. A movement direction M2 of the second limitation member 90b is the tangential direction of the fixing gear 66. In the second limitation member 90b, teeth 91b are provided side by side in the tangential direction of the fixing gear 66, and the second limitation member 90b functions as a so-called rack gear. Moreover, the gear apparatus is provided with a stopper 120 for stopping the movement of the second limitation member 90b in the state where the second limitation member 90b is engaged with the fixing gear 66. When the second limitation member 90b is engaged with the fixing gear 66, the phase of the tip of the tooth 91a of the first limitation member 90a corresponds to the phase of the root between the external teeth 67 of the fixing gear 66.

When the limitation members 90 are engaged with the fixing gear 66, the second limitation member 90b is moved toward the fixing gear 66 in the tangential direction of the fixing gear 66 ahead of the first limitation member 90a. When the tooth 91b of the second limitation member 90b contact the external tooth 67 of the fixing gear 66, force in the tangential direction of the fixing gear 66 acts on the external tooth 67 of the fixing gear 66 from internal tooth 90b of the second limitation member 90b, the fixing gear 66 is rotated, and the phase of the roots between the external teeth 67 of the fixing gear 66 sequentially corresponds to the phase of the tips of the internal teeth 90b of the second limitation member 90b, and thus the internal teeth 90b of the second limitation member 90b are engaged with the external teeth 67 of the fixing gear 66. When the second limitation member 90b is engaged with the fixing gear 66, the phase of the root between the external teeth 67 of the fixing gear 66 corresponds to the phase of the tip of the teeth 91a of the first limitation member 90a. Then, as the first limitation member 90a is moved toward the fixing gear 66 in the radial direction of the fixing gear 66, the first limitation member 90a is engaged with the fixing gear 66 without contact of the tip of the external teeth 67 of the fixing gear 66 and the tip of the teeth 91a of the first limitation member 90a.

## Claims

1. A gear apparatus comprising:
a spur gear (66) including teeth (67) and to be rotated around a rotation axis;
a rotation mechanism (44) to rotate the spur gear (66);
a limitation member (90) including tooth (91) to be engaged with the teeth (67) of the spur gear (66), the limitation member (90) to limit the rotation of the spur gear (66);
a movement mechanism (76, 84, 88) to move the limitation member (90) in the radial direction of the rotation axis such that the tooth (91) of the limitation member (90) is engaged with the teeth (67) of the spur gear (66), the movement mechanism (76, 84, 88) including a movement member (76);
an input mechanism to input power to actuate the movement mechanism (76, 84, 88), the input mechanism including an input member (78);
a transmission mechanism (101; 108; 114) to transmit the power input by the input mechanism (78) to the movement mechanism (76, 84, 88); and
an accumulation mechanism (101; 108; 114) provided in the transmission mechanism (101; 108; 114) and adapted to accumulate the power input by the input mechanism when a tip of the tooth (91) of the limitation member (90) contacts a tip of the tooth (67) of the spur gear (66), wherein the accumulation mechanism (101; 108; 114) includes an elastic member (101; 108; 114) provided between the input member (78) of the input mechanism and the movement member (76) of the movement mechanism (76, 84, 88),
**characterized in that**
the input member (78) of the input mechanism and the movement member (76) of the movement mechanism (76, 84, 88) are adapted to be rotated about a rotation axis which is coaxial with the rotation axis of the spur gear (66), and
the elastic member (101; 108; 114) is adapted to be deformed by the rotation of the input member (78) to produce urging force to rotate the movement member (76) in the same direction as the rotational direction of the input member (78).

2. The gear apparatus according to claim 1, wherein
the input member (78) has a shape of a plate substantially perpendicular to the rotation axis and includes a first receiving hole (96) penetrating the input member (78) and extending in a circumferential direction of the rotation axis, the movement member (76) has a shape of a plate substantially perpendicular to the rotation axis and includes a second receiving hole (96) penetrating the movement member (76) and extending in the circumferential direction of the rotation axis, the input member (78) and the movement member (76) are sequentially provided in a direction of the rotation axis such that, when the movement member (76) and the input member (78) are in phase, the first receiving hole (96) and the second receiving hole (96) are overlapped with each other, and the elastic member (101) is provided in the first receiving hole (96) and the second receiving hole (96).

3. The gear apparatus according to one of claims 1-2, further comprising:
a positioning mechanism (116, 118) to position the spur gear (66) so that the phase of a root between the teeth (67) of the spur gear (66) corresponds to the phase of the tip of the tooth (91) of the limitation member (90).

4. The gear apparatus according to claim 3, wherein
the positioning mechanism (116, 118) includes a lock member (118) to be locked between the teeth (67) of the spur gear (66), and an urging member (116) to urge the lock member (118) inwardly in the radial direction of the rotation axis.

5. An electric bending endoscope comprising:
a drive apparatus including a clutch mechanism (74);
a bending portion (24) to be bent by the drive apparatus; and
a gear apparatus according to any one of claims 1 to 4 provided in the drive apparatus.

## Patentansprüche

1. Getriebevorrichtung mit:
einem Stirnrad (66), das Zähne (67) aufweist und um eine Drehachse zu drehen ist;
einem Drehmechanismus (44) zum Drehen des Stirnrads (66);
einem Begrenzungselement (90), das einen Zahn (91) aufweist, der mit den Zähnen (67) des Stirnrads (66) in Eingriff zu bringen ist, wobei das Begrenzungselement (90) zum Begrenzen der Drehung des Stirnrads (66) vorgesehen ist;
einem Bewegungsmechanismus (76, 84, 88) zum Bewegen des Begrenzungselements (90) in der radialen Richtung der Drehachse derart, dass der Zahn (91) des Begrenzungselements (90) mit den Zähnen (67) des Stirnrads (66) in Eingriff kommt, wobei der Bewegungsmechanismus (76, 84, 88) ein Bewegungselement (76) aufweist;
einem Eingabemechanismus zum Eingeben von Energie, um den Bewegungsmechanismus (76, 84, 88) zu betätigen, wobei der Eingabemechanismus ein Eingabeelement (78) aufweist;
einem Übertragungsmechanismus (101; 108; 114) zum Übertragen der durch den Eingabemechanismus (78) eingegebenen Energie zum Bewegungsmechanismus (76, 84, 88); und
einem Speichermechanismus (101; 108; 114), der im Übertragungsmechanismus (101; 108; 114) vorgesehen ist und die durch den Eingabemechanismus eingegebene Energie zu speichern vermag, wenn eine Spitze des Zahns (91) des Begrenzungselements (90) mit einer Spitze des Zahns (67) des Stirnrads (66) in Kontakt ist, wobei der Speichermechanismus (101; 108; 114) ein elastisches Element (101; 108; 114) aufweist, das zwischen dem Eingabeelement (78) des Eingabemechanismus und dem Bewegungselement (76) des Bewegungsmechanismus (76, 84, 88) vorgesehen ist,
**dadurch gekennzeichnet, dass**
das Eingabeelement (78) des Eingabemechanismus und das Bewegungselement (76) des Bewegungsmechanismus (76, 84, 88) dazu ausgebildet sind, um eine Drehachse gedreht zu werden, die koaxial zur Drehachse des Stirnrads (66) verläuft, und
das elastische Element (101; 108; 114) dazu ausgebildet ist, durch die Drehung des Eingabeelements (78) so verformt zu werden, dass es eine Presskraft erzeugt, um das Bewegungselement (76) in dieselbe Richtung wie die Drehrichtung des Eingabeelements (78) zu drehen.

2. Getriebevorrichtung nach Anspruch 1, wobei
das Eingabeelement (78) die Form einer zur Drehachse im Wesentlichen senkrechten Platte besitzt und eine erste Aufnahmeöffnung (96) aufweist, die das Eingabeelement (78) durchdringt und sich in einer Umfangsrichtung der Drehachse erstreckt, das Bewegungselement (76) die Form einer zur Drehachse im Wesentlichen senkrechten Platte besitzt und eine zweite Aufnahmeöffnung (96) aufweist, die das Bewegungselement (76) durchdringt und sich in der Umfangsrichtung der Drehachse erstreckt, wobei das Eingabeelement (78) und das Bewegungselement (76) so nacheinander in einer Richtung der Drehachse vorgesehen sind, dass, wenn sich das Bewegungselement (76) und das Eingabeelement (78) in Phase befinden, die erste Aufnahmeöffnung (96) und die zweite Aufnahmeöffnung (96) einander überlappen, und das elastische Element (101) in der ersten Aufnahmeöffnung (96) und der zweiten Aufnahmeöffnung (96) vorgesehen ist.

3. Getriebevorrichtung nach einem der Ansprüche 1 bis 2, die des Weiteren aufweist:
einen Positionierungsmechanismus (116, 118), um das Stirnrad (66) so zu positionieren, dass die Phase eines Fußes zwischen den Zähnen (67) des Stirnrads (66) der Phase des Kopfs des Zahnes (91) des Begrenzungselements (90) entspricht.

4. Getriebevorrichtung nach Anspruch 3, wobei
der Positionierungsmechanismus (116, 118) ein Arretierelement (118), das zwischen den Zähnen (67) des Stirnrads (66) zu arretieren ist, und ein Presselement (116) aufweist, um das Arretierelement (118) nach innen in der Radialrichtung der Drehachse zu pressen.

5. Elektrisches Biege-Endoskop mit:
einer Antriebsvorrichtung, die einen Kupplungsmechanismus (74) aufweist;
einem Biegebereich (24) zum Biegen durch die Antriebsvorrichtung; und
einer Getriebevorrichtung nach einem der Ansprüche 1 bis 4, die in der Antriebsvorrichtung vorgesehen ist.

## Revendications

1. Appareil d'engrenage comprenant :
une roue droite cylindrique (66) comprenant des dents (67) et destinée à être mise en rotation autour d'un axe de rotation ;
un mécanisme de rotation (44) pour faire tourner la roue droite cylindrique (66) ;
un élément de limitation (90) comprenant une dent (91) destinée à être mise en prise avec les dents (67) de la roue droite cylindrique (66), l'élément de limitation (90) étant destiné à limiter la rotation de la roue droite cylindrique (66) ;
un mécanisme de déplacement (76, 84, 88) pour déplacer l'élément de limitation (90) dans la direction radiale de l'axe de rotation d'une manière telle que la dent (91) de l'élément de limitation (90) est en prise avec les dents (67) de la roue droite cylindrique (66), le mécanisme de déplacement (76, 84, 88) comprenant un élément de déplacement (76) ;
un mécanisme d'entrée destiné à délivrer en entrée une puissance pour actionner le mécanisme de déplacement (76, 84, 88), le mécanisme d'entrée comprenant un élément d'entrée (78) ;
un mécanisme de transmission (101 ; 108 ; 114) pour transmettre la puissance délivrée en entrée par le mécanisme d'entrée (78) au mécanisme de déplacement (76, 84, 88) ; et
un mécanisme d'accumulation (101 ; 108 ; 114) prévu dans le mécanisme de transmission (101 ; 108 ; 114) et adapté pour accumuler la puissance délivrée en entrée par le mécanisme d'entrée lorsqu'une pointe des dents (91) de l'élément de limitation (90) contacte une pointe de la dent (67) de la roue droite cylindrique (66), dans lequel le mécanisme d'accumulation (101 ; 108 ; 114) comprend un élément élastique (101 ; 108 ; 114) prévu entre l'élément d'entrée (78) du mécanisme d'entrée et l'élément de déplacement (76) du mécanisme de déplacement (76, 84, 88),
**caractérisé en ce que**
l'élément d'entrée (78) du mécanisme d'entrée et l'élément de déplacement (76) du mécanisme de déplacement (76, 84, 88) sont adaptés pour être mis en rotation autour d'un axe de rotation qui est coaxial à l'axe de rotation de la roue droite cylindrique (66), et
l'élément élastique (101 ; 108 ; 114) est adapté pour être déformé par la rotation de l'élément d'entrée (78) pour produire une force de sollicitation pour faire tourner l'élément de déplacement (76) dans le même sens que le sens de rotation de l'élément d'entrée (78).

2. Appareil d'engrenage selon la revendication 1, dans lequel
l'élément d'entrée (78) présente une forme d'une plaque sensiblement perpendiculaire à l'axe de rotation et comprend un premier trou de réception (96) pénétrant l'élément d'entrée (78) et s'étendant dans une direction circonférentielle de l'axe de rotation, l'élément de déplacement (76) présente une forme d'une plaque sensiblement perpendiculaire à l'axe de rotation et comprend un deuxième trou de réception (96) pénétrant l'élément de déplacement (76) et s'étendant dans la direction circonférentielle de l'axe de rotation, l'élément d'entrée (78) et l'élément de déplacement (76) sont séquentiellement prévus dans une direction de l'axe de rotation d'une manière telle que, lorsque l'élément de déplacement (76) et l'élément d'entrée (78) sont en phase, le premier trou de réception (96) et le deuxième trou de réception (96) se chevauchent l'un l'autre, et l'élément élastique (101) est prévu dans le premier trou de réception (96) et le deuxième trou de réception (96).

3. Appareil d'engrenage selon l'une quelconque des revendications 1 à 2, comprenant en outre :
un mécanisme de positionnement (116, 118) pour positionner la roue droite cylindrique (66) de sorte que la phase d'une racine entre les dents (67) de la roue droite cylindrique (66) correspond à la phase de la pointe de la dent (91) de l'élément de limitation (90).

4. Appareil d'engrenage selon la revendication 3, dans lequel le mécanisme de positionnement (116, 118) comprend un élément de blocage (118) destiné à être bloqué entre les dents (67) de la roue droite cylindrique (66), et un élément de sollicitation (116) pour solliciter l'élément de blocage (118) vers l'intérieur dans la direction radiale de la rotation.

5. Endoscope à courbure électrique comprenant :
un appareil d'entraînement comprenant un mécanisme d'embrayage (74) ;
une partie de courbure (24) destinée à être courbée par l'appareil d'entraînement ; et
un appareil d'engrenage selon l'une quelconque des revendications 1 à 4 prévu dans l'appareil d'entraînement.
